(19) Europäisches Patentamt
European Patent Office
Office européen des brevets

(11) Veröffentlichungsnummer: **0 402 486 A1**

(12) # EUROPÄISCHE PATENTANMELDUNG veröffentlicht nach Art. 158 Abs. 3 EPÜ

(21) Anmeldenummer: **90901096.9**

(51) Int. Cl.5: **C07C 211/52, C07C 209/10**

(22) Anmeldetag: **22.12.89**

(86) Internationale Anmeldenummer:
**PCT/SU89/00332**

(87) Internationale Veröffentlichungsnummer:
**WO 90/07489 (12.07.90 90/16)**

(30) Priorität: **30.12.88 SU 4622747**

(43) Veröffentlichungstag der Anmeldung:
**19.12.90 Patentblatt 90/51**

(84) Benannte Vertragsstaaten:
**CH DE GB IT LI**

(71) Anmelder: **ANDRIEVSKY, Alexandr Mikhailovich**
**ul. Sivashskaya, 4-3-100**
**Moscow, 113149(SU)**

Anmelder: **GORELIK, Mikhail Viktorovich**
**ul. Pervomaiskaya 46-60 Moskovskaya obl.**
**Dolgoprudny, 141700(SU)**

Anmelder: **AVIDON, Sergei Viktorovich**
**ul. Godovikova, 1-2-72**
**Moscow, 129085(SU)**

Anmelder: **GORDIEVSKAYA, Evgenia Vasilievna**
**ul. Presnensky Val, 42-7**
**Moscow, 123557(SU)**

Anmelder: **ALTMAN, Elena Shulimovna**
**Petrovsko-Razumovsky proezd, 7-14**
**Moscow 125083(SU)**

Anmelder: **VOROZHTSOV, Georgy Nikolaevich**
**ul. Sadovaya-Spasskaya, 21-208**
**Moscow, 107078(SU)**

Anmelder: **DJUMAEV, Kirill Mikhailovich**
**ul. B. Filevskaya, 55-2-27**
**Moscow, 121433(SU)**

(72) Erfinder: **ANDRIEVSKY, Alexandr Mikhailovich**
**ul. Sivashskaya, 4-3-100**
**Moscow, 113149(SU)**
Erfinder: **GORELIK, Mikhail Viktorovich**
**ul. Pervomaiskaya 46-60 Moskovskaya obl.**
**Dolgoprudny, 141700(SU)**
Erfinder: **AVIDON, Sergei Viktorovich**
**ul. Godovikova, 1-2-72**
**Moscow, 129085(SU)**
Erfinder: **GORDIEVSKAYA, Evgenia Vasilievna**
**ul. Presnensky Val, 42-7**
**Moscow, 123557(SU)**
Erfinder: **ALTMAN, Elena Shulimovna**
**Petrovsko-Razumovsky proezd, 7-14**
**Moscow 125083(SU)**
Erfinder: **VOROZHTSOV, Georgy Nikolaevich**
**ul. Sadovaya-Spasskaya, 21-208**
**Moscow, 107078(SU)**
Erfinder: **DJUMAEV, Kirill Mikhailovich**
**ul. B. Filevskaya, 55-2-27**
**Moscow, 121433(SU)**

(74) Vertreter: **von Füner, Alexander, Dr. et al**
**Patentanwälte v. Füner, Ebbinghaus, Finck**
**Mariahilfplatz 2 & 3**
**D-8000 München 90(DE)**

(54) **VERFAHREN ZUR HERSTELLUNG VON 2-BROM-4,6-DINITROANILIN.**

EP 0 402 486 A1

Xerox Copy Centre

(57) Die Herstellung von 2-Brom-4,6-dinitroanilin erfolgt durch Behandlung des 2-Brom-4,6-dinitrochlorbenzols mit wässeriger Ammoniaklösung in Gegenwart eines anionaktiven Dispergierungsmittels, die in einem wässerigen oder einem wässerig-organischen Medium bei einer Temperatur von 86 bis 90 °C vorgenommen wird.

# VERFAHREN ZUR HERSTELLUNG VON 2-BROM-4,6-DINITROANILIN

## Gebiet der Technik

Die vorliegende Erfindung bezieht sich auf organische Synthese und insbesondere betrifft sie ein Verfahren zur Herstellung von 2-Brom-4,6-Dinitroanilin.

## Zugrundeliegender Stand der Technik

Gegenwärtig sind bekannt Verfahren zur Herstellung von 2-Brom-4,6-dinitroanilin durch Bromieren des 2,4-Dinitroanilins mit Molekularbrom, das gemäß CS, A, 152603 vorgenommen wird, in Essigsäure in Gegenwart von Schwefel; oder gemäß DE 3014972, in einem wässerigen Medium in Gegenwart von Wasserstoffperoxid oder gemäß Journal für chemische Industrie, Nr. 11, 1986, S. 399, in verdünnter Salzsäure in Gegenwart von Natriumhypochlorit vorgenommen wird.

In allen diesen bekannten Verfahren, in denen die Ammonolyse nicht die letzte Reaktionsstufe darstellt, ist in der Reaktionsmasse als Beimengung 2,4-Dinitroanilin vorhanden.

Ebenfalls bekannt ist die Herstellung von 2-Brom-4,6-dinitroanilin durch Ammonolyse des 2-Brom-4,6-dinitrochlorbenzols, die wie folgt realisiert wird: man läßt das gasförmige Ammoniak durch den trockenen (nichtverdünnten) Alkohol durch und behandelt man mit dieser Lösung die Ausgangsverbindung (Joiurnal Chemical Society 125, 2482-84, 1924).

Abgesehen von der erreichten hohen Ausbeute an Endprodukt, ~ 85%, hat das genannte Verfahren keine großtechnische Anwendung gefunden und wurde lediglich in der Laborpraxis infolge dessen angewendet, weil es notwendig war, komplizierte technologische Bedingungen zu erfüllen, das gasförmige Ammoniak soll durch eine Säule mit festem KOH zwecks Trocknung durchgelassen werden und der Alkohol soll abdestilliert werden. Hierdurch verursacht die Anwendung des gasförmigen trockenen Ammoniaks aus einem Ballon und des wasserfreien Alkohols in dem genannten Verfahren besonders kategorische Bedingungen, die eine solche Produktion unrentabel machen.

Bekannt ist auch die Herstellung von 2-Brom-4,6-dinitroanilin durch Ammonolyse des 2-4-Dinitrochlorbenzols

mit wässerigem Ammoniak, die bei einer Temperatur von 100°C in einem Autoklaven erfolgt, und durch darauffolgendes Bromieren der Reaktionsmasse (US, A, 4381409).

Im Ergebnis der Realisierung des genannten Verfahrens weist die Reaktionsmasse als Beimengung 2-4-Dinitroanilin und harzhaltige Verbindungen auf.

Offenbarung der Erfindung

Der vorliegenden Erfindung liegt die Aufgabe zugrunde, durch die Prozeßführung unter den Bedingungen, die eine vorwiegende Substitution lediglich eines Chloratoms gegen eine Aminogruppe im Benzolring bewirken, ein Verfahren zur Herstellung von 2-Brom-4,6-dinitroanilin zu entwickeln, welches es ermöglicht, ein reineres Produkt zu gewinnen.

Die genannte Aufgabe wird dadurch gelöst, daß im Verfahren zur Herstellung von 2-Brom-4,6-dinitroanilin, das durch Behandlung des Derivates des Dinitrobenzols mit Ammoniak unter Anfallen des Endproduktes erfolgt, man, erfindungsgemäß, als Derivate des Dinitrochlorbenzols das 2-Brom-4,6-dinitrochlorbenzol verwendet, und seine Behandlung mit Ammoniak in Gegenwart eines anionaktiven Dispergierungsmittels im wässerigen Medium oder wässerig-organischem Medium bei einer Temperatur von 86 bis 90°C erfolgt.

Die Ausbeute an Endprodukt beträgt 90% der Theorie.

Zweckmäßigerweise, soll man, erfindungsgemäß, als Dispergierungsmittel das Methylen-bis-(naphthalin-sulfonat) dinatrium verwenden.

Weitere Ziele und Vorteile der angemeldeten Erfindung werden aus der nachstehenden ausführlichen Beschreibung des Verfahrens zur Herstellung von 2-Brom-4,6-dinitroanilin und aus den konkreten Beispielen für die Ausführung dieses Verfahrens ersichtlich.

Beste Ausführungsvariante der Erfindung

Das erfindungsgemäße Verfahren zur Herstellung von 2-Brom-4,6-dinitroanilin besteht in der Ammonolyse des 2-Brom-4,6-dinitrochlorbenzols.

Die Ausgangsverbindung, 2-Brom-4,6-dinitrochlorbenzol,

kann in beliebigem bekanntem Verfahren, beispielsweise, vorzugsweise gewonnen werden: aus Chlorbenzol, das vorher mit einem Gemisch aus Salpeter- und Schwefelsäure behandelt wird, oder aus individuellen o-,p-Nitrochlorbenzolen oder aus eutektischem Gemisch der o-, p-, m-Nitrochlorbenzolen, die man der Behandlung mit Brom oder mit Alkalimetallbromid, mit Salpetersäure oder mit Alkalimetall--nitrat und mit Schwefelsäure oder mit Oleum bei folgenden Molverhältnissen der Reagenzien, wie das genannte aromatische Halogenderivat-Verbindung: Brom oder Alkalimetallbromid: Schwefelsäure oder Oleum gleich 1,0:0,5-1,5 oder 1,0-3,0: 2,0-4,0 oder 2,5-5,0: 6,0-70,0 bei einer Temperatur von 20 bis 120°C ausgesetzt werden.

Erfindungsgemäß wird die Ammonolyse unter milden Bedingungen bei einer Temperatur von 86 bis 90°C in einem wässerigen Medium oder in einem organischen Lösungsmittel, beispielsweise, im Chlorbenzol in Gegenwart eines anionaktiven Dispergierungsmittels durchgeführt:

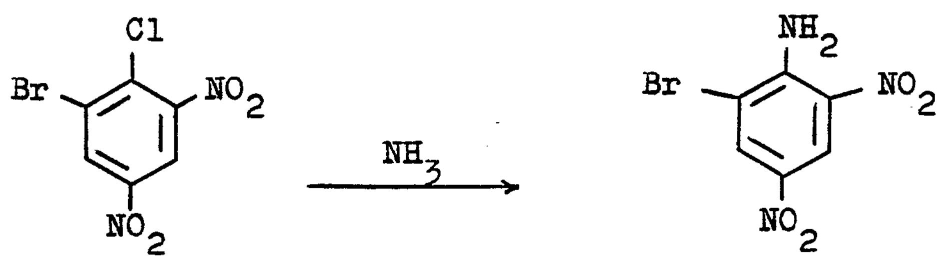


Die Ammonolyse führt man unter Verwendung einer wässerigen Ammoniaklösung, die eine Konzentration von 30% aufweist.

Die selektive Substitution eines Chloratoms gegen eine Aminogruppe, und nicht eines Bromatoms oder Chloratoms und Bromatoms gleichzeitig, kam zustande infolge der Prozeßführung in Gegenwart eines beliebigen bekannten anionaktiven Dispergierungsmittels, beispielsweise, Methylen-bis (naphthalinsulfonat) dinatriums oder eines Kondensationsproduktes des Kresol-Formaldehyd-Harzes mit 2-6-Natriumnaphthylsulfonat, mit Formaldehyd oder mit Natriumsulfid.

Die Durchführung der Ammonolyse unter den genannten Bedingungen in Gegenwart eines Dispergierungsmittels schließt außerdem die Entstehung neben dem Endprodukt auch

harzhaltiger Beimengungen und des 2-Brom-4,6-dinitrophenols in der Reaktionsmasse.

Die Reaktionsmasse der gemäß dem beanspruchten Verfahren durchgeführten Ammonolyse enthält nur das Endprodukt und bedeutend weniger als 0,5% Bromchlordinitrobenzol.

Zur besseren Erläuterung der vorliegenden Erfindung werden nachstehende Beispiele für ihre konkrete Ausführung angeführt.

Beispiel 1

2,8 g 2-Brom-4,6-dinitrochlorbenzol werden in 100 ml 30%iger Ammoniaklösung untergebracht, man setzt 0,28 g Methylen-bis-(naphthalinsulfomat) dinatrium zu und vermischt man bei einer Temperatur von 86 bis 90° innerhalb von 8 Stunden.

Die Beendigung der Reaktion ermittelt man im Dünnschichtchromatografie-Verfahren. Die Reaktionsmasse wird abgekühlt, der Niederschlag des 2-Brom-4,6-dinitroanilins wird abgefiltert, mit Wasser gewaschen und getrocknet. Man erhält 2,2 g 2-Brom-4,6-dinitroanilin in einer Ausbeute von 85% der Theorie; Schmelzpunkt beträgt von 149 bis 151°C.

Beispiel 2

2,8 g 2-Brom-4,6-dinitrochlorbenzol unterbringt man in 30 ml Chlorbenzol, vermischt man bis zur vollständigen Auflösung, setzt man 100 ml 30%ige Ammoniaklösung und 0,28 g Methylen-bis-(naphthalinsulfonat) dinatrium zu und vermischt man bei einer Temperatur von 88 bis 90°C innerhalb von 8 Stunden. Nach der Beendigung der Reaktion (die im Dünnschichtchromatografie-Verfahren ermittelt wird) setzt man der Reaktionsmasse weitere 30 ml Wasser zu und destilliert man das Chlorbenzol zusammen mit Wasser ab. Der ausgefallene Niederschlag wird abgefiltert und getrocknet. Man erhält 2,35 g 2-Brom-4,6-dinitroanilin. Die Ausbeute beträgt 90% der Theorie, Schmelzpunkt beträgt von 150 bis 151°C.

Beispiel 3

28 g 2-Brom-4,6-dinitrochlorbenzol unterbringt man in 300 ml 30%iger wässeriger Ammoniaklösung, setzt man 3 g Methylen-bis-(naphthalinsulfonat)dinatrium zu und vermischt

man innerhalb von 8 Stunden bei einer Temperatur von 86 bis 90°C.

Die Beendigung der Reaktion ermittelt man im Dünschichtchromatografie-Verfahren.

Die Reaktionsmasse wird abgekühlt, der Niederschlag des 2-Brom-4,6-dinitroanilins wird abgefiltert, mit Wasser gewaschen und getrocknet. Die Ausbeute an Produkt beträgt 22,5 g (85,3% der Theorie).

Beispiel 4

28 g 2-Brom-4,6-dinitrochlorbenzol unterbringt man in 300 ml 30%iger Ammoniaklösung, man setzt 3 g eines Kondensationsproduktes des Kresol-Formaldehyd-Harzes mit 2,6-Natriumnaphtholsulfonat, mit Formaldehyd und Natriumsulfid zu, im weiteren verläuft die Reaktion analog der in Beispiel 3 beschriebenen.

Man erhält 32 g 2-Brom-4,6-dinitroanilin in einer Ausbeute von 85% der Theorie).

Gewerbliche Andendbarkeit

Die Erfindung wird bei der Produktion von dispersen Farbstoffen anwendung finden.

## PATENTANSPRÜCHE

1. Verfahren zur Herstellung von 2-Brom-4,6-dinitroanilin durch Behandlung des Dinitrochlorbenzol-Derivates mit wässeriger Ammoniaklösung unter Anfallen des Endproduktes, dadurch gekennzeichnet, daß man als Dinitrochlorbenzol-Derivate 2-Brom-4,6-dinitrochlorbenzol verwendet und seine Behandlung mit Ammoniak in Gegenwart eines anionaktiven Dispergierungsmittels in einem wässerigen Medium oder einem wässerig-organischen Medium bei einer Temperatur von 86 bis 90°C durchführt.

2. Verfahren nach Anspruch 1, dadurch gekennzeichnet, daß man als anionaktives Dispergierungsmittel Methylen-bis-(naphthalinsulfonat) dinatrium verwendet.

# INTERNATIONAL SEARCH REPORT

International Application No PCT/SU 89/00332

**I. CLASSIFICATION OF SUBJECT MATTER** (if several classification symbols apply, indicate all) [6]

According to International Patent Classification (IPC) or to both National Classification and IPC

IPC[5] C07C 211/52, 209/10

**II. FIELDS SEARCHED**

Minimum Documentation Searched [7]

| Classification System | Classification Symbols |
|---|---|
| IPC[4] | C07C 87/60, 85/04 |

Documentation Searched other than Minimum Documentation to the Extent that such Documents are Included in the Fields Searched [8]

**III. DOCUMENTS CONSIDERED TO BE RELEVANT** [9]

| Category [*] | Citation of Document, [11] with indication, where appropriate, of the relevant passages [12] | Relevant to Claim No [13] |
|---|---|---|
| A | DE, A1, 3014972 (CHEMISCHE FABRIK KALK GmbH), 22 October 1981 see the claims, (cited in the description) | 1,2 |
| A | Journal of the Chemical Society, vol.125, N° 12 1924 (GURNEY AND JACKSON, London), Shrirang M. Sane et al, "Behaviour of Nitrophenols with p-Toluenesulphonyl Chloride", pags 2481-2484 (cited in the description) | 1,2 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 12 March 1990 (12.03.90) | 2 April 1990 (02.04.90) |
| **International Searching Authority** | **Signature of Authorized Officer** |
| ISA/SU | |